(19) Europäisches Patentamt
European Patent Office
Office européen des brevets

(11) EP 4 152 260 A1

(12) EUROPEAN PATENT APPLICATION
published in accordance with Art. 153(4) EPC

(43) Date of publication:
22.03.2023 Bulletin 2023/12

(51) International Patent Classification (IPC):
G06T 7/62 (2017.01)        G06T 7/11 (2017.01)
G06T 11/20 (2006.01)

(21) Application number: 21825617.0

(22) Date of filing: 16.06.2021

(86) International application number:
PCT/KR2021/007532

(87) International publication number:
WO 2021/256846 (23.12.2021 Gazette 2021/51)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 16.06.2020 KR 20200072958

(71) Applicant: Deep Bio Inc.
Seoul 08380 (KR)

(72) Inventors:
• MUN, Ui Geo
  Gwangmyeong-si, Gyeonggi-do 14305 (KR)
• CHO, Min Ah
  Seoul 04776 (KR)
• KWAK, Tae Yeong
  Seoul 05119 (KR)
• KIM, Sun Woo
  Seongnam-si, Gyeonggi-do 13599 (KR)

(74) Representative: Flügel Preissner Schober Seidel
Patentanwälte PartG mbB
Nymphenburger Straße 20
80335 München (DE)

(54) METHOD FOR MEASURING LENGTH OF LIVING TISSUE INCLUDED IN SLIDE IMAGE, AND COMPUTING SYSTEM FOR PERFORMING SAME

(57) Disclosed are a method for measuring the length of a living tissue included in a slide image, and a computing system for performing same. According to one aspect of the present invention, provided is the method for measuring the length of a living tissue included in a slide image, the method comprising the steps of segmenting, by a computing system, the slide image into a plurality of patches having a predetermined size; generating, by the computing system, a graph corresponding to the slide image, wherein the graph comprises a node corresponding to each patch including the living tissue from among the plurality of patches and, when the living tissue spans two arbitrary patches adjacent to each other in a vertical, horizontal, or diagonal direction, two nodes corresponding to the two patches adjacent to each other are connected via an edge; for each edge included in the graph, setting a weight of the edge; for each connected component of the graph including two or more nodes, detecting shortest paths between all node pairs included in the connected components and determining a longest shortest path having the longest length from among the detected shortest paths between all the node pairs; and calculating the length of the living tissue included in the slide image, on the basis of the longest shortest path of each connected component constituting the graph.

FIG. 1

| Input slide image | S100 |
| Divide slide image into plurality of patches | S110 |
| Detect living tissue and/or lesion tissue from slide image | S120 |
| Generate graph corresponding to slide image | S130 |
| Set weight for each edge of graph | S140 |
| Determine longest shortest path for each connected component in graph | S150 |
| Calculate length of living tissue included in slide image | S160 |

EP 4 152 260 A1

**Description**

Technical Field

The present disclosure relates to a method for measuring the length of a living tissue included in a slide image and a computing system for performing the same.

Background Art

One of the major tasks performed by pathology or department of pathology is to read a patient's biometric image (e.g., a tissue slide of the patient) to perform a diagnosis to determine the condition or symptom for a specific disease. This diagnosis is a method that depends on the experience and knowledge of skilled medical personnel for a long time. A recent trend is to gradually increase a method of reading a slide image generated by digital imaging instead of a living tissue slide.

Meanwhile, in most diagnostic practices, pathologists use a microscope and a measuring instrument (e.g., ruler) to manually measure tissue size on slide images, and on the slide image, the living tissue is not in the form of a straight line, but in the form of a very irregular curve, and as a result, there is a problem in that the size of the tissue may be measured differently depending on the pathologist who measures the tissue, and even when the same pathologist measures the same tissue, the size of the tissue may vary for each measurement. In other words, although the size of a living tissue or a lesion tissue in which a lesion appears is treated as a very important factor in pathological diagnosis (e.g., prostate cancer biopsy) through a living body slide image, it is very difficult to accurately measure the size of the tissue.

Disclosure of the Invention

Technical Goals

Accordingly, the present disclosure has been proposed to solve the problems of the related art as described above, and an object of the present disclosure is to provide a method for accurately and objectively measuring the length of a living tissue included in a slide image.

Technical Solutions

According to an aspect of the present disclosure, there is provided a method for measuring a length of a living tissue included in a slide image including dividing, by a computing system, the slide image into a plurality of patches having a predetermined size, wherein each of the plurality of patches is any one obtained by dividing the slide image into an N×M grid, where N and M are each an integer of 2 or more; generating, by the computing system, a graph corresponding to the slide image, wherein the graph includes nodes corresponding to each patch including a living tissue among the plurality of patches, and when the living tissue spans any two patches adjacent to each other in a vertical, horizontal, or diagonal direction, two nodes corresponding to the two adjacent patches are connected by an edge; for each edge included in the graph, setting a weight of the edge; for each connected component of the graph including two or more nodes, detecting shortest paths between all node pairs included in the connected component and determining a longest shortest path with a longest length among the shortest paths between all node pairs; and calculating the length of the living tissue included in the slide image based on the longest shortest path of each connected component included in the graph.

In an embodiment, the setting of the weight of the edge may include calculating the weight W of the edge by the following [Equation 1].

$$[\text{Equation 1}]$$

$$W = A / \{E(v1) \times E(v2)\}$$

(where v1 and v2 are two nodes connected by the edge,

A is 1 when the patches corresponding to nodes v1 and v2, respectively, are vertically or horizontally adjacent, and $\sqrt{2}$ when diagonally adjacent,

and E(x) is the number of edges connected to node x on the graph)

In an embodiment, at least some of living tissues included in the slide image may be lesion tissues, and the setting of the weight of the edge may include calculating the weight W of the edge by the following [Equation 2].

[Equation 2]

$$W = A / [\{E(v1) \times E(v2)\} \times \{C(v1) \times C(v2)\}]$$

(where v1 and v2 are two nodes connected by the edge,

A is 1 when the patches corresponding to nodes v1 and v2, respectively, are vertically or horizontally adjacent, and $\sqrt{2}$ when diagonally adjacent,

E(x) is the number of edges connected to node x on the graph,

and C(y) is 1 when the patch corresponding to node y on the graph does not contain lesion tissue, and $\alpha$ when it does (where $\alpha$ is a predetermined real number greater than 1))

In an embodiment, the method may include binarizing the plurality of patches; and for each of the plurality of binarized patches, based on whether a pixel representing a living tissue exists at each edge line and each corner of the binarized patch, determining whether a living tissue spans a patch adjacent to the patch.

In an embodiment, at least some of living tissues included in the slide image may be lesion tissues, and the method may further include generating a tissue mask in which a living tissue region included in the slide image is masked; generating a lesion mask in which the lesion tissue included in the slide image is masked; and determining whether the living tissue or lesion tissue is included in the plurality of patches, based on the tissue mask and the lesion mask.

According to another aspect of the present disclosure, there is provided a method for measuring a length of a lesion tissue included in a slide image, including dividing, by a computing system, the slide image into a plurality of patches having a predetermined size, wherein each of the plurality of patches is any one obtained by dividing the slide image into an N×M grid, where N and M are each an integer of 2 or more; generating, by the computing system, a graph corresponding to the slide image, wherein the graph includes nodes corresponding to each patch including a lesion tissue among the plurality of patches, and when the lesion tissue spans any two patches adjacent to each other in a vertical, horizontal, or diagonal direction, two nodes corresponding to the two adjacent patches are connected by an edge; for each edge included in the graph, setting a weight of the edge; for each connected component of the graph including two or more nodes, detecting shortest paths between all node pairs included in the connected component and determining a longest shortest path with a longest length among the shortest paths between all node pairs; and calculating the length of the lesion tissue included in the slide image based on the longest shortest path of each connected component included in the graph.

According to another aspect of the present disclosure, a recorded computer program installed in a data processing device for performing the method described above is provided.

According to another aspect of the present disclosure, a computing system including a processor; and a memory configured to store a computer program, wherein the computer program, when executed by the processor, causes the computing system to perform the method described above is provided.

According to another aspect of the present disclosure, there is provided a computing system for performing a method for measuring a length of a living tissue included in a slide image, including a dividing module configured to divide the slide image into a plurality of patches having a predetermined size, wherein each of the plurality of patches is any one obtained by dividing the slide image into an N×M grid, where N and M are each an integer of 2 or more; a graph generating module configured to generate a graph corresponding to the slide image, wherein the graph includes nodes corresponding to each patch including a living tissue among the plurality of patches, and when the living tissue spans any two patches adjacent to each other in a vertical, horizontal, or diagonal direction, two nodes corresponding to the two adjacent patches are connected by an edge; a weight setting module configured to, for each edge included in the graph, set a weight of the edge; a shortest path determining module configured to, for each connected component of the graph including two or more nodes, detect shortest paths between all node pairs included in the connected component and determine a longest shortest path with a longest length among the shortest paths between all node pairs; and a calculating module configured to calculate the length of the living tissue included in the slide image based on the longest shortest path of each connected component included in the graph.

In an embodiment, the weight setting module may calculate the weight W of the edge by the following [Equation 3].

[Equation 3]

$$W = A / \{E(v1) \times E(v2)\}$$

(where v1 and v2 are two nodes connected by the edge,

A is 1 when the patches corresponding to nodes v1 and v2, respectively, are vertically or horizontally adjacent, and $\sqrt{2}$ when diagonally adjacent,
and E(x) is the number of edges connected to node x on the graph)

In an embodiment, at least some of living tissues included in the slide image may be lesion tissues, and the weight setting module may calculate the weight W of the edge by the following [Equation 4].

[Equation 4]

$$W = A / [\{E(v1) \times E(v2)\} \times \{C(v1) \times C(v2)\}]$$

(where v1 and v2 are two nodes connected by the edge,

A is 1 when the patches corresponding to nodes v1 and v2, respectively, are vertically or horizontally adjacent, and $\sqrt{2}$ when diagonally adjacent,
E(x) is the number of edges connected to node x on the graph,
and C(y) is 1 when the patch corresponding to node y on the graph does not contain lesion tissue, and $\alpha$ when it does (where $\alpha$ is a predetermined real number greater than 1))

In an embodiment, the computing system may further include an image processing module configured to binarize the plurality of patches; and a determining module configured to, for each of the plurality of binarized patches, based on whether a pixel representing a living tissue exists at each edge line and each corner of the binarized patch, determine whether a living tissue spans a patch adjacent to the patch.

In an embodiment, at least some of living tissues included in the slide image may be lesion tissues, and the computing system may further include an image processing module configured to generate a tissue mask in which a living tissue region included in the slide image is masked, and generate a lesion mask in which the lesion tissue included in the slide image is masked; and a determining module configured to determine whether the living tissue or lesion tissue is included in the plurality of patches, based on the tissue mask and the lesion mask.

According to another aspect of the present disclosure, there is provided a computing system for performing a method for measuring a length of a lesion tissue included in a slide image, including a dividing module configured to divide the slide image into a plurality of patches having a predetermined size, wherein each of the plurality of patches is any one obtained by dividing the slide image into an N×M grid, where N and M are each an integer of 2 or more; a graph generating module configured to generate a graph corresponding to the slide image, wherein the graph includes nodes corresponding to each patch including a lesion tissue among the plurality of patches, and when the lesion tissue spans any two patches adjacent to each other in a vertical, horizontal, or diagonal direction, two nodes corresponding to the two adjacent patches are connected by an edge; a weight setting module configured to, for each edge included in the graph, set a weight of the edge; a shortest path determining module configured to, for each connected component of the graph including two or more nodes, detect shortest paths between all node pairs included in the connected component and determine a longest shortest path with a longest length among the shortest paths between all node pairs; and a calculating module configured to calculate the length of the lesion tissue included in the slide image based on the longest shortest path of each connected component included in the graph.

Advantageous Effects

According to the technical idea of the present disclosure, it is possible to provide a method for accurately and objectively measuring the length of a living tissue or a lesion tissue included in a slide image.

Brief Description of Drawings

In order to more fully understand the drawings recited in the detailed description of the disclosure, a brief description of each drawing is provided.

FIG. 1 is a flowchart illustrating an example of a living tissue length measurement method according to an embodiment of the present disclosure.

FIGS. 2A to 2G are diagrams for explaining a specific process of performing a living tissue length measurement method according to an embodiment of the present disclosure.

FIG. 3 is a diagram for explaining a method for generating, by a computing system, an edge of a graph according to an embodiment of the present disclosure.

FIG. 4 is a diagram for explaining an example in which a generated graph includes two or more connected components.

FIG. 5 is a diagram for explaining embodiments of setting a weight of each edge included in a graph.

FIG. 6 is a block diagram illustrating a schematic configuration of a computing system according to an embodiment of the present disclosure.

FIGS. 7A to 7E are diagrams illustrating an example of a process in which a computing system measures the length of a living tissue included in a living body slide image according to an embodiment of the present disclosure.

Best Mode for Carrying Out the Invention

Since the present disclosure can apply various transformations and can have various embodiments, specific embodiments are illustrated in the drawings and described in detail in the detailed description. However, this is not intended to limit the present disclosure to specific embodiments, and should be understood to include all modifications, equivalents, and substitutes included in the spirit and scope of the present disclosure. In describing the present disclosure, if it is determined that a detailed description of a related known technology may obscure the gist of the present disclosure, the detailed description thereof will be omitted.

Terms such as first, second, etc., may be used to describe various elements, but the elements should not be limited by the terms. The above terms are used only for the purpose of distinguishing one component from another.

The terms used in the present application are only used to describe specific embodiments, and are not intended to limit the present disclosure. The singular expression includes the plural expression unless the context clearly dictates otherwise.

In the present specification, it should be understood that the terms such as "comprises" or "have" are intended to designate that the features, numbers, steps, operations, components, parts, or combinations thereof described in the specification exist, and this does not preclude the possibility of addition or existence of one or more other features or numbers, steps, operations, components, parts, or combinations thereof.

In addition, in the present specification, when any one component 'transmits' data to another component, the component may directly transmit the data to the other component, or may transmit the data to the other component through at least one other component. Conversely, when one component 'directly transmits' data to another component, it means that the data is transmitted from the component to the other component without passing through the other component.

Hereinafter, preferred embodiments of the present disclosure will be described in detail with reference to the accompanying drawings. Like reference numerals in each drawing indicate like elements.

A computing system according to an embodiment of the present disclosure may be an information processing device. A computing system 100 may be a processing system such as a desktop computer, a laptop computer, or a server, and may be a processing device including a handheld device such as a mobile phone, a satellite phone, a wireless phone, a session initiation protocol (SIP), a wireless local loop (WLL) station, a smart phone, a tablet PC, and a personal digital assistant (PDA).

The computing system may perform a method for measuring the length of a living tissue included in a slide image (hereinafter, referred to as a 'living tissue length measurement method').

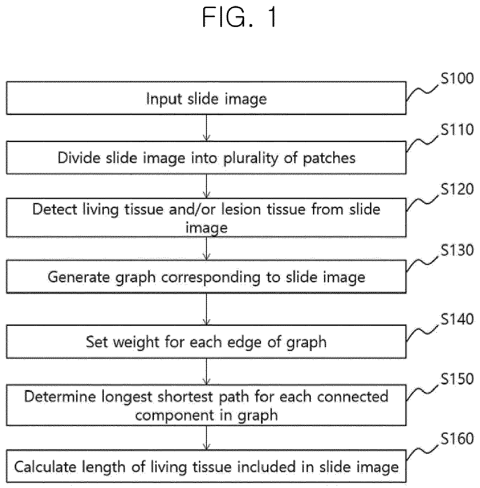

FIG. 1 is a flowchart illustrating an example of a living tissue length measurement method performed by the computing system.

The computing system may receive a slide image S100. The slide image may be an image obtained by photographing a living tissue. For example, the slide image may be a pathological slide image obtained by photographing a living tissue to determine the presence or absence of a lesion of a predetermined disease (e.g., cancer).

The computing system may divide the slide image into a plurality of patches having a predetermined size S110. Each of the plurality of patches may be any one obtained by dividing the slide image into an $N \times M$ grid (where N and M are each an integer of 2 or more).

Each of the plurality of patches may be in the form of a square having the same size.

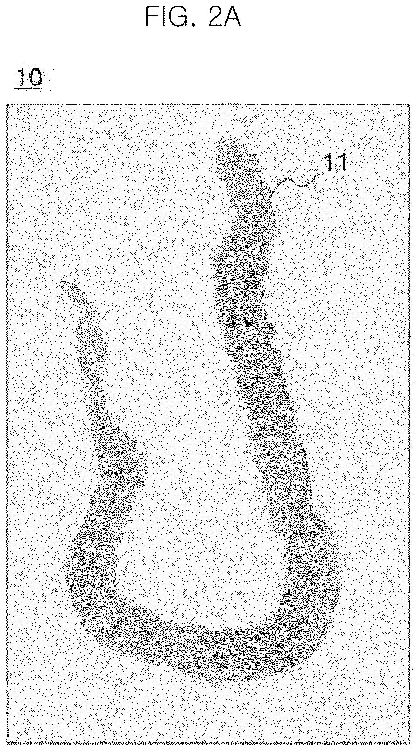

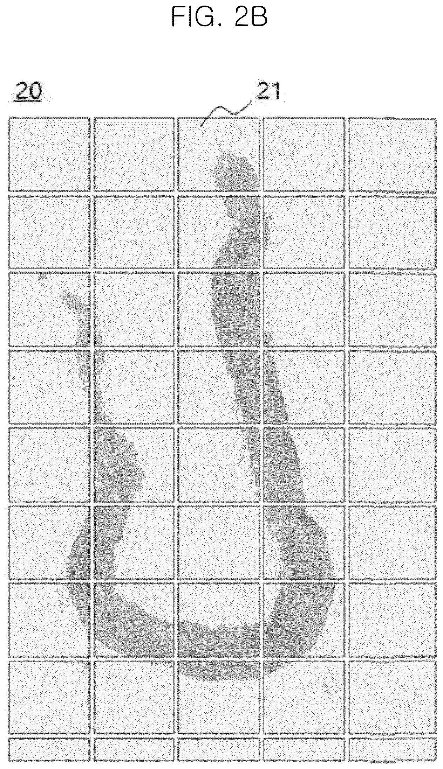

FIG. 2A is a diagram illustrating an example of a slide image, and FIG. 2B is a diagram illustrating an example in which the slide image of FIG. 2A is divided into patches. When the computing system 100 receives the slide image 10 as shown in FIG. 2A, the dividing module 110 may form 40 patches (e.g., 21) by dividing them into a grid shape 20 of 5×8 as shown in FIG. 2B. Each of the slide patches (e.g., 21) may have the same size.

The computing system may detect a living tissue from the slide image, and according to an embodiment, may detect a lesion tissue, which is a living tissue including a lesion caused by a predetermined disease, from the slide image S120. In the present specification, detecting the living tissue included in the image may mean detecting a region in which the living tissue is photographed in the image or determining a patch including the region in which the living tissue is photographed among a plurality of patches forming the image, and detecting the lesion tissue included in the image may mean detecting a region in which the lesion tissue is photographed in the image or determining a patch in which the lesion tissue is photographed among a plurality of patches forming the image.

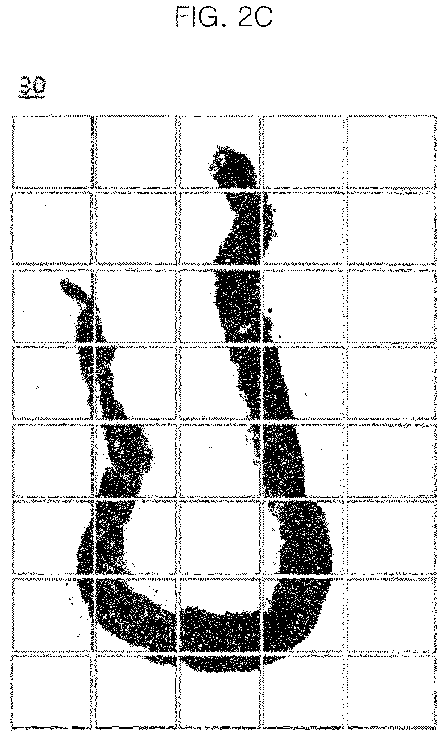

In an embodiment, the computing system may detect a living tissue included in the slide image through image binarization, which is a computer vision-related technology. In more detail, the computing system may perform pre-processing such as noise filtering and/or median blurring on the slide image, convert it to gray scale, and perform binarization by performing thresholding on the converted image and dividing each pixel based on a predetermined threshold. According to an embodiment, the computing system may perform binarization for each patch obtained by dividing the slide image, and the result of binarizing the patches 20 of FIG. 2B in this way is shown in FIG. 2C.

In addition to this, there may be various embodiments in which the computing system detects a living tissue. For example, the computing system may detect the living tissue included in the slide image by using a pre-learned artificial intelligence network to detect the living tissue.

Meanwhile, the computing system may detect the lesion tissue from the slide image using the above-described binarization technique or a pre-learned artificial intelligence network.

Referring back to FIG. 1, the computing system may generate a graph (particularly, an undirected graph) corresponding to the slide image S130.

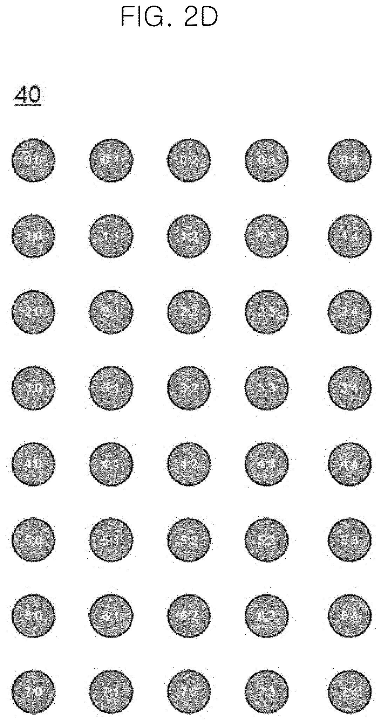

More specifically, the computing system may generate nodes of the graph. The computing system may generate nodes corresponding to each patch included in the slide image S131. FIG. 2D is a diagram illustrating nodes 40 corresponding to each of the patches 20 of FIG. 2B.

The computing system may also generate edges of the graph. In the computing system, when a living tissue spans any two patches adjacent to each other in a vertical, horizontal, or diagonal direction, two nodes corresponding to the two adjacent patches may be connected by an edge.

For example, the computing system may scan upper/lower/left/right edge lines of the patch to determine whether a living tissue spans a patch adjacent to the patch. In other words, the computing system may scan each edge line of the patch, and when pixels representing living tissue are formed on at least a part of the corresponding edge line, may determine that adjacent patches that share the corresponding edge and the corresponding patch spans living tissue.

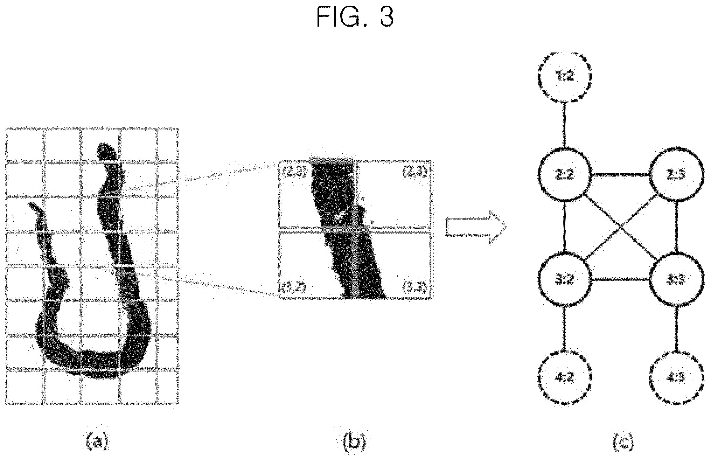

FIG. 3 is a diagram for explaining a method for the computing system to generate edges of a graph. FIG. 3A shows each patch of the slide image, FIG. 3B shows the (2, 2)th patch, (2,3)th patch, (3,2)th patch, and (3,3)th patch which are some of the patches of FIG. 3A, and FIG. 3C shows the node corresponding to FIG. 3B and the edge of each node generated by the computing system.

In the example of FIG. 3B, in the (2, 2)th patch, since pixels (indicated by thick lines) representing living tissues are formed on the upper, lower, and right edge lines, the computing system may determine that the living tissue spans the (2, 2)th patch and the (1, 2)th patch adjacent to the top, the (2, 2)th patch and the (3, 2)th patch adjacent to the bottom, the (2, 2)th patch and the (2, 3)th patch adjacent to the right, and as shown in FIG. 3C, may connect the node (2:2) and the node (1:2) as an edge, the node (2:2) and the node (3:2) as an edge, and the node (2:2) and the node (2:3) as an edge.

In addition, when pixels representing living tissues are formed at each corner of the patch and a diagonally adjacent corner facing it, the computing system may determine that the living tissue spans the two patches.

In the example of FIG. 3B, since pixels representing living tissues are formed in the lower right corner of the (2, 2)th patch and the upper left corner of the (3, 3)th patch facing it, as shown in FIG. 3B, the computing system may connect the node (2:2) corresponding to the (2, 2)th patch and the node (3:3) corresponding to the (3, 3)th patch as an edge.

In a similar manner, the computing system may determine the edges of the node (2:3), node (3:2), and node (3:3).

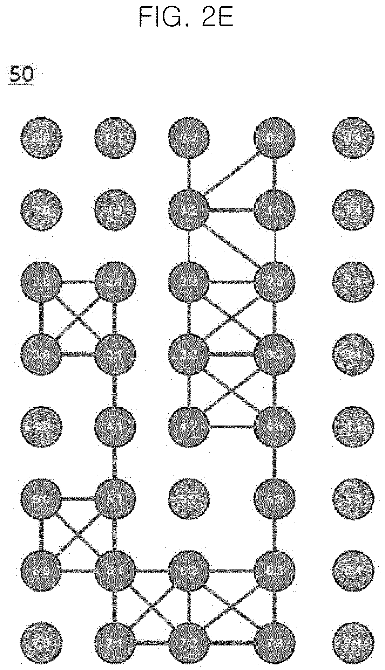

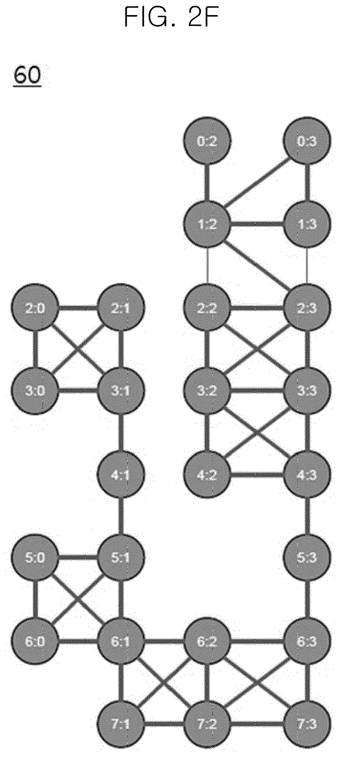

A graph 50 in which the nodes of FIG. 3D are connected by edges in the same manner as described above is shown in FIG. 2E. Meanwhile, the computing system may generate a graph corresponding to the slide by removing the independent node that is not connected to any other nodes, and the graph 60 from which the independent node is removed from the graph of FIG. 2E is shown in FIG. 2F.

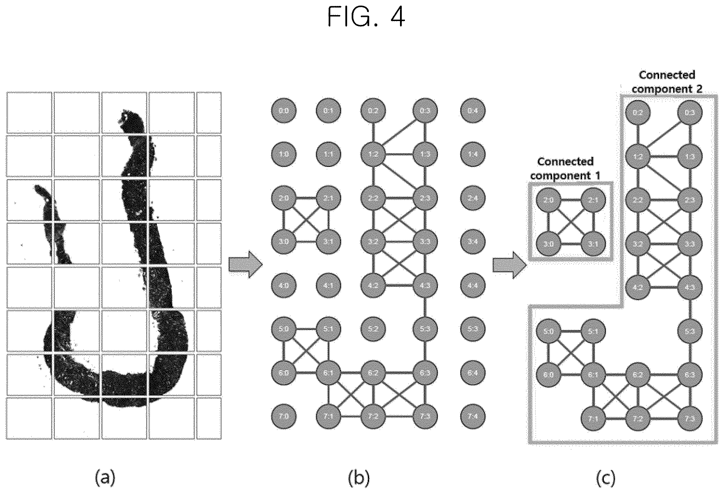

Meanwhile, depending on the slide image, the graph generated by the computing system may include two or more connected components. If the slide image is composed of patches as shown in FIG. 4A, the computing system may generate a graph including two connected components as shown in FIG. 4C by removing an independent node from the graph as shown in FIG. 4B.

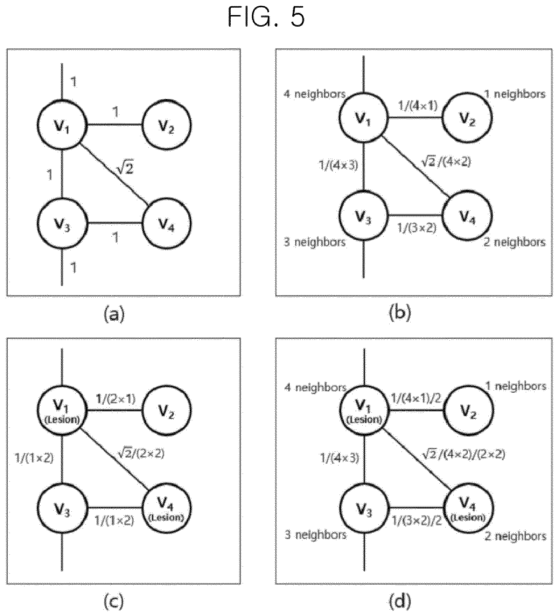

Referring back to FIG. 1, the computing system may set a weight for each edge included in the graph S140, which will be described with reference to FIG. 5. In the example of FIG. 5, it is assumed that the arrangement of each node is the same as that of the corresponding patch.

In an embodiment, the computing system may set the weight to 1 in the case of an edge connecting nodes corresponding to adjacent patches in the vertical, horizontal directions, and in the case of an edge connecting nodes corresponding to diagonally adjacent patches, may set the weight to $\sqrt{2}$ , an example of which is shown in FIG. 5A. As shown in FIG. 5A, edges (V1, V2), (V1, V3), (V3, V4) have a weight of 1 since the edges connect nodes corresponding to the vertically or horizontally adjacent patches, and since the edge <V1, V4> connects the nodes corresponding to the adjacent patches in the diagonal direction, the weight is set to $\sqrt{2}$ .

In another embodiment, the computing system may set the weight by further considering the number of neighboring nodes of each of the two nodes connected by the edge (here, the neighboring node is a node connected to the corresponding node through the edge, and the number of the neighboring node is equal to the number of edges the corresponding node has). This is to allow the longest shortest path to be described later to pass through the middle of the living tissue as much as possible. In a weight setting method in consideration of the number of neighboring nodes, the weight W of the edge may be determined by the following [Equation 1], an example of which is shown in FIG. 5B.

$$[\text{Equation 1}]$$

$$W = A \: / \: \{E(v1) \times E(v2)\}$$

(where v1 and v2 are two nodes connected by the edge,

A is 1 when the patches corresponding to nodes v1 and v2, respectively, are vertically or horizontally adjacent, and $\sqrt{2}$ when diagonally adjacent,

and E(x) is the number of edges connected to node x (i.e., number of the neighboring node of node v))

As shown in FIG. 5B, assuming that node V1 has 4 neighboring nodes (i.e., 4 edges), node V2 has 1 neighboring node, node V3 has 3 neighboring nodes and node V4 has 2 neighboring nodes, each edge of FIG. 5B may have the following weight.

- $$\text{Weight of (V1, V2)} = 1 \: / \{E(V1) \times E(V2)\} = 1/(4 \times 1) = 1/4$$

- $$\text{Weight of (V1, V3)} = 1 \: / \{E(V1) \times E(V3)\} = 1/(4 \times 3) = 1/12$$

- $$\text{Weight of (V1, V4)} = \sqrt{2} \: / \{E(V1) \times E(V4)\} = \sqrt{2}/(4 \times 2) = \sqrt{2}/8$$

- $$\text{Weight of (V3, V4)} = 1 \: / \{E(V3) \times E(V4)\} = 1/(3 \times 2) = 1/6$$

Meanwhile, when at least a part of the living tissue included in the slide image is lesion tissue, the computing system may set a weight by further considering whether a patch corresponding to two nodes connected by an edge includes lesion tissue. This is because, when the living tissue includes a lesion tissue, the length of the living tissue including the lesion tissue must be measured.

In a weight setting method considering whether or not a patch includes a lesion tissue, the weight W of the edge may be determined by the following [Equation 2], an example of which is shown in FIG. 5C.

$$[\text{Equation 2}]$$

$$W = A / \{C(v1)*C(v2)\}$$

(where v1 and v2 are two nodes connected by the edge,

A is 1 when the patches corresponding to nodes v1 and v2, respectively, are vertically or horizontally adjacent, and $\sqrt{2}$ when diagonally adjacent,

and C(y) is 1 when the patch corresponding to any node v on the graph does not contain lesion tissue, and $\alpha$ when it does (where $\alpha$ is a predetermined real number greater than 1, and $\alpha$ is assumed to be 2 below))

As shown in FIG. 5C, when lesion tissues are included in nodes V1 and V4, each edge of FIG. 5C may have the following weight.

■

$$\text{Weight of } (V1, V2) = 1 /\{C(V1) \times C(V2)\} = 1/(2 \times 1) = 1/2$$

■

$$\text{Weight of } (V1, V3) = 1 /\{C(V1) \times C(V3)\} = 1/(1 \times 2) = 1/2$$

■

$$\text{Weight of } (V1, V4) = \sqrt{2} /\{C(V1) \times C(V4)\} = \sqrt{2} /(2 \times 2) = \sqrt{2} /4$$

■

$$\text{Weight of } (V3, V4) = 1 /\{C(V3) \times C(V4)\} = 1/(1 \times 2) = 1/2$$

Meanwhile, in another embodiment, the computing system may set the weight in consideration of both the number of neighboring nodes and whether the patch includes lesion tissue. In this case, the computing system may determine the weight W of the edge by the following [Equation 4], an example of which is shown in FIG. 5D.

$$[\text{Equation 4}]$$

$$W = A / \{E(v1) \times E(v2)\} / \{C(v1) \times C(v2)\}$$

Nodes V1 and V4 include lesion tissue, and if the number of neighboring nodes of each node is the same as in FIG. 5D, each edge of FIG. 5D may have the following weight.

■

$$\text{Weight of } (V1, V2) = 1/\{E(V1) \times E(V2)\}/\{C(V1) \times C(V2)\} = 1/(4 \times 1)/(2 \times 1) = 1 /8$$

■

$$\text{Weight of (V1, V3)} = 1/\{E(V1)\times E(V3)\}/\{C(V1)\times C(V3)\} = 1/(4\times3)/(1\times2) = 1/24$$

■

$$\text{Weight of (V1, V4)} = \sqrt{2}/\{E(V1)\times E(V2)\}/\{C(V1)\times C(V4)\} = \sqrt{2}/(4\times2)/(2\times2) = \sqrt{2}/32$$

■

$$\text{Weight of (V3, V4)} = 1/\{E(V1)\times E(V2)\}/\{C(V3)\times C(V4)\} = 1/(3\times2)/(1\times2) = 1/12$$

Referring back to FIG. 1, the computing system may, for each connected component of the graph including two or more nodes, detect a shortest path between all node pairs included in the connected component and determine a longest shortest path with a longest length among the shortest paths between all node pairs.

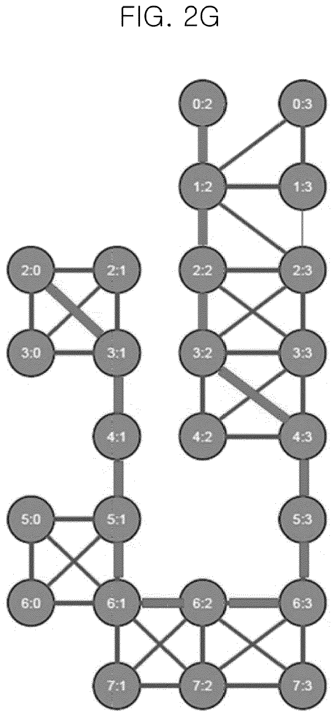

In an embodiment, the computing system may determine the longest shortest path using a Floyd-Warshall algorithm. In other words, the computing system may calculate the shortest path between each pair of nodes included in the connected component, and determine the longest shortest path that is the shortest path having the longest distance among them. FIG. 2G is a diagram illustrating the longest shortest path of the graph shown in FIG. 2F.

Referring back to FIG. 1, the computing system may calculate the length of the living tissue included in the slide image based on the longest shortest path of each of the connected components included in the graph.

In an embodiment, the computing system may recalculate the length of each longest shortest path by setting the lengths of all edges on the longest shortest path of each connected component to 1 or $\sqrt{2}$, depending on the arrangement of patches corresponding to two nodes connected by the corresponding edges, and then multiply the recalculated length by the actual distance between the vertically or horizontally adjacent patches to calculate the actual length of the living tissue.

In an embodiment, the computing system may sum all the distances of the longest shortest paths of each connected component, calculate the total length of the living tissue based on the summed value, and output it to the outside of the computing system. In another embodiment, the computing system may calculate the length of each piece included in the living tissue included in the slide image based on the distances of the longest shortest paths of all the connected components, and output it to the outside of the computing system.

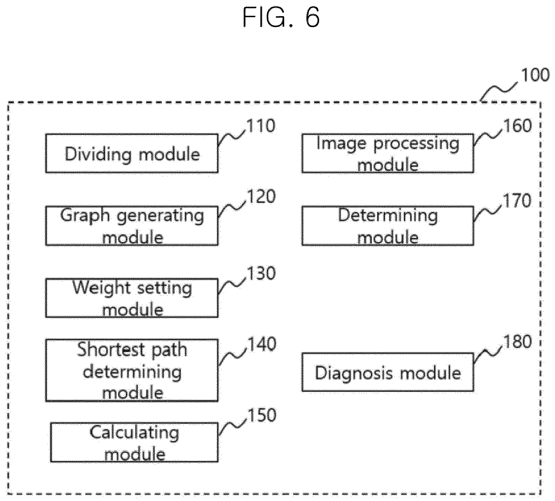

FIG. 6 is a block diagram illustrating a schematic configuration of the computing system 100 according to an embodiment of the present disclosure.

Referring to FIG. 6, the computing system 100 may include a dividing module 110, a graph generating module 120, a weight setting module 130, a shortest path determining module 140, and a calculating module 150. In addition, the computing system 100 may further include an image processing module 160, a determining module 170, and/or a diagnosis module 180. According to an embodiment, the computing system 100 may include only some of the components illustrated in FIG. 6, or may include more components than this. For example, the computing system 100 may further include at least one communication module capable of communicating with an external system through a network or a control module capable of controlling functions and/or resources of other components included in the computing system 100, a storage module capable of storing various information, an input/output module capable of interfacing with the user and receiving information from the outside or outputting information to the outside, and the like.

The computing system 100 may include hardware resources and/or software necessary to implement the technical idea of the present disclosure, and does not necessarily mean one physical component or one device. In other words, the computing system 100 may mean a logical combination of hardware and/or software provided to implement the technical idea of the present disclosure, and if necessary, may be implemented as a set of logical configurations for implementing the technical idea of the present disclosure by being installed in devices spaced apart from each other and performing respective functions. In addition, the computing system 100 may refer to a set of components separately implemented for each function or role for implementing the technical idea of the present disclosure.

In this specification, a module may refer to a functional and structural combination of hardware for carrying out the technical idea of the present disclosure and software for driving the hardware. For example, it can be easily deduced to an average expert in the art of the present disclosure that the module may refer to a logical unit of a predetermined code and a hardware resource for executing the predetermined code, and does not necessarily mean a physically connected code or one type of hardware.

The dividing module 110 may divide the slide image into a plurality of patches having a predetermined size. Here, each of the plurality of patches may be any one obtained by dividing the slide image into an N×M grid (N and M are each an integer of 2 or more), and may have a square shape of the same size.

At least a part of the living tissue included in the slide image may be a lesion tissue, and the diagnosis module 180 may determine whether a lesion due to a predetermined disease exists in the slide image or each of the plurality of patches. Alternatively, the diagnosis module 180 may detect a lesion tissue in the slide image or each of the plurality of patches.

In an embodiment, the image processing module 160 may binarize the plurality of patches, and the determining module 170 may, for each of the plurality of binarized patches, determine whether a living tissue spans a patch adjacent to the patch, based on whether a pixel representing a living tissue exists at each edge line and each corner of the binarized patch.

In another embodiment, the image processing module 160 may generate a tissue mask in which the living tissue region included in the slide image is masked, and generate a lesion mask in which the lesion tissue included in the slide image is masked, and the determining module may determine whether the living tissue or lesion tissue is included in the plurality of patches, based on the tissue mask and the lesion mask.

Meanwhile, the graph generating module 120 may generate a graph corresponding to the slide image. The graph generated by the graph generating module 120 may include nodes corresponding to each patch including a living tissue among the plurality of patches, and when a living tissue spans any two patches adjacent to each other in the vertical, horizontal, or diagonal direction, two nodes corresponding to the two adjacent patches may be connected by an edge.

The weight setting module 130 may set a weight of each edge included in the graph.

In an embodiment, the weight setting module 130 may calculate the weight W of the edge by the following [Equation 5].

$$[\text{Equation 5}]$$

$$W = A \,/\, \{E(v1) \times E(v2)\}$$

(where v1 and v2 are two nodes connected by the edge,

A is 1 when the patches corresponding to nodes v1 and v2, respectively, are vertically or horizontally adjacent, and $\sqrt{2}$ when diagonally adjacent, and

E(x) is the number of edges connected to node x on the graph)

In another embodiment, the weight setting module may calculate the weight W of the edge by the following [Equation 6].

$$[\text{Equation 6}]$$

$$W = A \,/\, [\{E(v1) \times E(v2)\} \times \{C(v1) \times C(v2)\}]$$

(where v1 and v2 are two nodes connected by the edge,

A is 1 when the patches corresponding to nodes v1 and v2, respectively, are vertically or horizontally adjacent, and $\sqrt{2}$ when diagonally adjacent,

E(x) is the number of edges connected to node x on the graph, and

C(y) is 1 when the patch corresponding to node y on the graph does not include lesion tissue, and $\alpha$ when it does (where $\alpha$ is a predetermined real number greater than 1))

Meanwhile, the shortest path determining module 140 may, for each connected component of the graph including two or more nodes, detect shortest paths between all node pairs included in the connected component and determine a longest shortest path with a longest length among the shortest paths between all node pairs, and the calculating module 150 may calculate the length of the living tissue included in the slide image based on the longest shortest path of each of the connected components included in the graph.

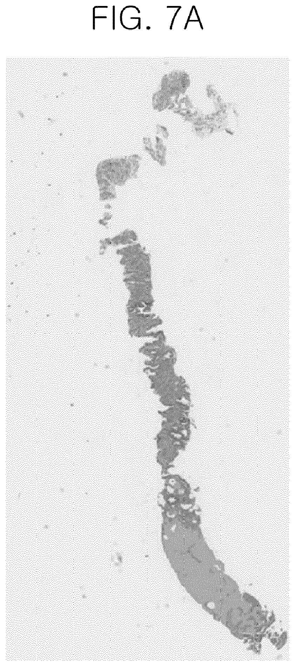

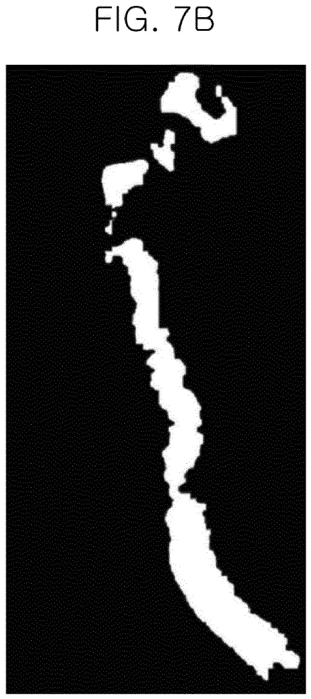

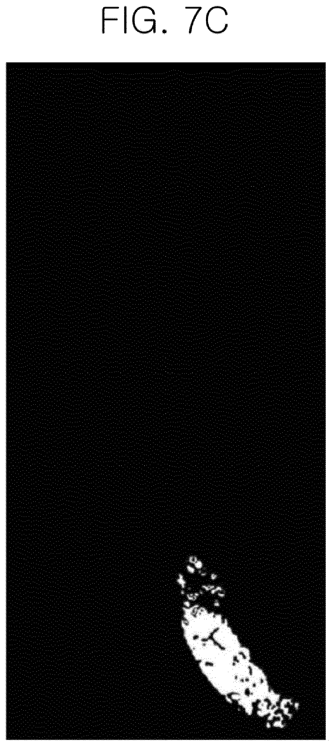

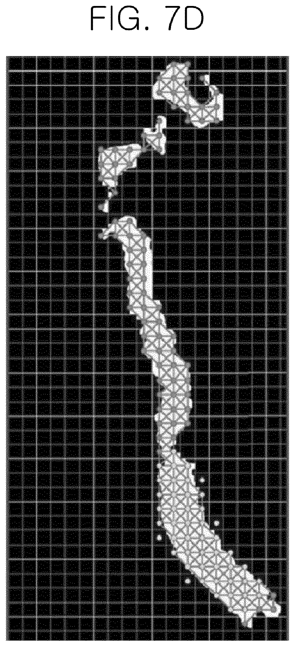

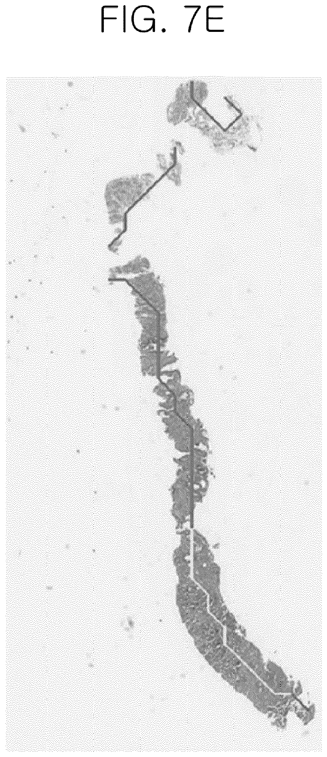

FIG. 7 is a diagram illustrating an example of a process in which the computing system measures the length of a living tissue included in a living body slide image.

When a slide image as shown in FIG. 7A is input, as shown in FIG. 7B, the computing system 100 may generate a tissue mask in which the living tissue region included in the slide image is masked, and as shown in FIG. 7C, may generate a lesion mask in which the lesion tissue included in the slide image is masked.

The computing system 100 may divide the slide image into a plurality of patches and generate a graph corresponding to the slide image as shown in FIG. 7D.

Thereafter, the computing system may determine the longest shortest path of each connected component in the graph of FIG. 7D. In FIG. 7E, the longest shortest path of each connected component is displayed overlapped on the slide image.

Meanwhile, the above-described method for measuring the length of a living tissue may be applied as a method for measuring the length of a lesion tissue very easily. In other words, in order to perform the method for measuring the length of a lesion tissue according to an embodiment of the present disclosure, the computing system may divide the slide image into a plurality of patches having a predetermined size (where each of the plurality of patches is any one obtained by dividing the slide image into an N×M grid, and N and M are each an integer of 2 or more).

The computing system may generate a graph corresponding to the slide image. Here, the graph may include a node corresponding to each of the patches including the lesion tissue among the plurality of patches, and when the lesion tissue spans any two patches adjacent to each other in a vertical, horizontal, or diagonal direction, two nodes corresponding to the two adjacent patches may be connected by an edge.

In addition, the computing system may set a weight of each edge included in the graph. Here, the computing system may set the weight of the corresponding edge in consideration of the number of neighboring nodes of the two nodes connected by the edge, which has been described above.

The computing system may, for each connected component of the graph including two or more nodes, detect shortest paths between all node pairs included in the connected component and determine a longest shortest path with a longest length among the shortest paths between all node pairs.

The computing system may calculate the length of the lesion tissue included in the slide image based on the longest shortest path of each of the connected components included in the graph.

Meanwhile, according to an embodiment, the computing system 100 may include at least one processor and a memory for storing a program executed by the processor. The processor may include a single-core CPU or a multi-core CPU. The memory may include high-speed random access memory and may include non-volatile memory such as one or more magnetic disk storage devices, flash memory devices, or other non-volatile solid-state memory devices. Access to memory by the processor and other components may be controlled by a memory controller.

Meanwhile, the method for measuring the length of a living tissue/lesion tissue according to an embodiment of the present disclosure may be implemented in the form of a computer readable program instruction and stored in a computer readable recording medium. The computer readable recording medium includes all types of recording devices in which data readable by a computer system is stored.

The program instructions recorded on the recording medium may be specially designed and configured for the present disclosure, or may be known and available to those skilled in the software field.

Examples of the computer readable recording medium include magnetic media such as hard disks, floppy disks and magnetic tapes, optical media such as CD-ROMs and DVDs, magneto-optical media such as floptical disks and hardware devices specially configured to store and execute program instructions, such as ROM, RAM, flash memory, and the like. In addition, the computer readable recording medium is distributed in a computer system connected through a network, so that the computer readable code may be stored and executed in a distributed manner.

Examples of the program instruction include not only machine code such as generated by a compiler, but also a device for electronically processing information using an interpreter or the like, for example, a high-level language code that may be executed by a computer.

The hardware devices described above may be configured to operate as one or more software modules to perform the operations of the present disclosure, and vice versa.

The above description of the present disclosure is for illustration, and those skilled in the art to which the present disclosure pertains will understand that it may be easily modified into other specific forms without changing the technical spirit or essential characteristics of the present disclosure. Therefore, it should be understood that the embodiments described above are illustrative in all respects and not restrictive. For example, each component described as a single type may be implemented in a distributed manner, and likewise components described as distributed may be implemented in a combined form.

The scope of the present disclosure is indicated by the following claims rather than the above detailed description, and all changes or modifications derived from the meaning and scope of the claims and their equivalents should be construed as being included in the scope of the present disclosure.

Industrial Applicability

Method for measuring the length of a living tissue included in a slide image and a computing system for performing the same

**Claims**

1. A method for measuring a length of a living tissue included in a slide image, comprising:

   dividing, by a computing system, the slide image into a plurality of patches having a predetermined size, wherein each of the plurality of patches is any one obtained by dividing the slide image into an N×M grid, where N and M are each an integer of 2 or more;
   generating, by the computing system, a graph corresponding to the slide image, wherein the graph includes nodes corresponding to each patch including a living tissue among the plurality of patches, and when the living tissue spans any two patches adjacent to each other in a vertical, horizontal, or diagonal direction, two nodes corresponding to the two adjacent patches are connected by an edge;
   for each edge included in the graph, setting a weight of the edge;
   for each connected component of the graph including two or more nodes, detecting shortest paths between all node pairs included in the connected component and determining a longest shortest path with a longest length among the shortest paths between all node pairs; and
   calculating the length of the living tissue included in the slide image based on the longest shortest path of each connected component included in the graph.

2. The method according to claim 1, wherein the setting the weight of the edge comprises:

   calculating the weight W of the edge by the following [Equation 1]:

$$[\text{Equation 1}]$$

$$W = A \,/\, \{E(v1) \times E(v2)\}$$

   where v1 and v2 are two nodes connected by the edge,
   A is 1 when the patches corresponding to nodes v1 and v2, respectively, are vertically or horizontally adjacent, and $\sqrt{2}$ when diagonally adjacent, and
   E(x) is the number of edges connected to node x on the graph.

3. The method according to claim 1, wherein at least some of living tissues included in the slide image are lesion tissues, and
   the setting of the weight of the edge comprises:

   calculating the weight W of the edge by the following [Equation 2]:

$$[\text{Equation 2}]$$

$$W = A \,/\, [\{E(v1) \times E(v2)\} \times \{C(v1) \times C(v2)\}]$$

   where v1 and v2 are two nodes connected by the edge,

   A is 1 when the patches corresponding to nodes v1 and v2, respectively, are vertically or horizontally adjacent, and $\sqrt{2}$ when diagonally adjacent,

E(x) is the number of edges connected to node x on the graph, and

C(y) is 1 when the patch corresponding to node y on the graph does not contain lesion tissue, and $\alpha$ when it does (where $\alpha$ is a predetermined real number greater than 1).

4. The method according to claim 1, further comprising:

binarizing the plurality of patches; and

for each of the plurality of binarized patches, based on whether a pixel representing a living tissue exists at each edge line and each corner of the binarized patch, determining whether a living tissue spans a patch adjacent to the patch.

5. The method according to claim 1, wherein at least some of living tissues included in the slide image are lesion tissues, and

the method further comprises:

generating a tissue mask in which a living tissue region included in the slide image is masked;

generating a lesion mask in which the lesion tissue included in the slide image is masked; and

determining whether the living tissue or lesion tissue is included in the plurality of patches, based on the tissue mask and the lesion mask.

6. A method for measuring a length of a lesion tissue included in a slide image, comprising:

dividing, by a computing system, the slide image into a plurality of patches having a predetermined size, wherein each of the plurality of patches is any one obtained by dividing the slide image into an N×M grid, where N and M are each an integer of 2 or more;

generating, by the computing system, a graph corresponding to the slide image, wherein the graph includes nodes corresponding to each patch including a lesion tissue among the plurality of patches, and when the lesion tissue spans any two patches adjacent to each other in a vertical, horizontal, or diagonal direction, two nodes corresponding to the two adjacent patches are connected by an edge;

for each edge included in the graph, setting a weight of the edge;

for each connected component of the graph including two or more nodes, detecting shortest paths between all node pairs included in the connected component and determining a longest shortest path with a longest length among the shortest paths between all node pairs; and

calculating the length of the lesion tissue included in the slide image based on the longest shortest path of each connected component included in the graph.

7. A recorded computer program installed in a data processing device for performing the method according to claim 1 or 6.

8. A computing system comprising:

a processor; and

a memory configured to store a computer program,

wherein the computer program, when executed by the processor, causes the computing system to perform the method according to any one of claims 1 to 6.

9. A computing system for performing a method for measuring a length of a living tissue included in a slide image, comprising:

a dividing module configured to divide the slide image into a plurality of patches having a predetermined size, wherein each of the plurality of patches is any one obtained by dividing the slide image into an N×M grid, where N and M are each an integer of 2 or more;

a graph generating module configured to generate a graph corresponding to the slide image, wherein the graph includes nodes corresponding to each patch including a living tissue among the plurality of patches, and when the living tissue spans any two patches adjacent to each other in a vertical, horizontal, or diagonal direction, two nodes corresponding to the two adjacent patches are connected by an edge;

a weight setting module configured to, for each edge included in the graph, set a weight of the edge;

a shortest path determining module configured to, for each connected component of the graph including two or more nodes, detect shortest paths between all node pairs included in the connected component and determine a longest shortest path with a longest length among the shortest paths between all node pairs; and

a calculating module configured to calculate the length of the living tissue included in the slide image based on the longest shortest path of each connected component included in the graph.

10. The computing system according to claim 9, wherein the weight setting module calculates the weight W of the edge by the following [Equation 3]:

[Equation 3]

$$W = A / \{E(v1) \times E(v2)\}$$

where v1 and v2 are two nodes connected by the edge,

A is 1 when the patches corresponding to nodes v1 and v2, respectively, are vertically or horizontally adjacent, and $\sqrt{2}$ when diagonally adjacent, and

E(x) is the number of edges connected to node x on the graph.

11. The computing system according to claim 9, wherein at least some of living tissues included in the slide image are lesion tissues, and

the weight setting module calculates the weight W of the edge by the following [Equation 4]:

[Equation 4]

$$W = A / [\{E(v1) \times E(v2)\} \times \{C(v1) \times C(v2)\}]$$

where v1 and v2 are two nodes connected by the edge,

A is 1 when the patches corresponding to nodes v1 and v2, respectively, are vertically or horizontally adjacent, and $\sqrt{2}$ when diagonally adjacent,

E(x) is the number of edges connected to node x on the graph, and

C(y) is 1 when the patch corresponding to node y on the graph does not contain lesion tissue, and $\alpha$ when it does (where $\alpha$ is a predetermined real number greater than 1).

12. The computing system according to claim 9, further comprising:

an image processing module configured to binarize the plurality of patches; and

a determining module configured to, for each of the plurality of binarized patches, based on whether a pixel representing a living tissue exists at each edge line and each corner of the binarized patch, determine whether a living tissue spans a patch adjacent to the patch.

13. The computing system according to claim 9, wherein at least some of living tissues included in the slide image are lesion tissues, and

the computing system further comprises:

an image processing module configured to generate a tissue mask in which a living tissue region included in the slide image is masked, and generate a lesion mask in which the lesion tissue included in the slide image is masked; and

a determining module configured to determine whether the living tissue or lesion tissue is included in the plurality of patches, based on the tissue mask and the lesion mask.

**14.** A computing system for performing a method for measuring a length of a lesion tissue included in a slide image, comprising:

a dividing module configured to divide the slide image into a plurality of patches having a predetermined size, wherein each of the plurality of patches is any one obtained by dividing the slide image into an $N \times M$ grid, where N and M are each an integer of 2 or more;

a graph generating module configured to generate a graph corresponding to the slide image, wherein the graph includes nodes corresponding to each patch including a lesion tissue among the plurality of patches, and when the lesion tissue spans any two patches adjacent to each other in a vertical, horizontal, or diagonal direction, two nodes corresponding to the two adjacent patches are connected by an edge;

a weight setting module configured to, for each edge included in the graph, set a weight of the edge;

a shortest path determining module configured to, for each connected component of the graph including two or more nodes, detect shortest paths between all node pairs included in the connected component and determine a longest shortest path with a longest length among the shortest paths between all detected node pairs; and

a calculating module configured to calculate the length of the lesion tissue included in the slide image based on the longest shortest path of each connected component included in the graph.

# FIG. 1

Input slide image — S100

Divide slide image into plurality of patches — S110

Detect living tissue and/or lesion tissue from slide image — S120

Generate graph corresponding to slide image — S130

Set weight for each edge of graph — S140

Determine longest shortest path for each connected component in graph — S150

Calculate length of living tissue included in slide image — S160

# FIG. 2A

## FIG. 2B

FIG. 2C

30

# FIG. 2D

# FIG. 2E

FIG. 2F

60

# FIG. 2G

FIG. 3

FIG. 4

FIG. 5

(a)

(b)

(c)

(d)

FIG. 6

# FIG. 7A

# FIG. 7B

# FIG. 7C

# FIG. 7D

FIG. 7E

## INTERNATIONAL SEARCH REPORT

| International application No. |
| --- |
| **PCT/KR2021/007532** |

| A. | CLASSIFICATION OF SUBJECT MATTER |
| --- | --- |

G06T 7/62(2017.01)i; G06T 7/11(2017.01)i; G06T 11/20(2006.01)i

According to International Patent Classification (IPC) or to both national classification and IPC

| B. | FIELDS SEARCHED |
| --- | --- |

Minimum documentation searched (classification system followed by classification symbols)

G06T 7/62(2017.01); A61B 5/22(2006.01); A61B 6/03(2006.01); A63B 69/00(2006.01); G06T 1/00(2006.01); G06T 7/11(2017.01); G06T 7/60(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 슬라이드 이미지(slide image), 생체 조직(biological tissue), 노드(node), 연결 성분(connected component), 최단 경로(shortest path), 길이(length)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| A | KR 10-2015-0020830 A (THE ASAN FOUNDATION) 27 February 2015 (2015-02-27)<br>    See paragraph [0086]; claims 5 and 9; and figure 7. | 1-14 |
| A | 조용만 등. 척도 없는 네트워크를 위한 그래프 레이아웃 알고리즘. 정보과학회논문지 : 시스템 및 이론. vol. 34, no. 5, pp. 202-213, June 2007 (CHO, Yongman et al. A Graph Layout Algorithm for Scale-free Network. Journal of KIISE : Computer Systems and Theory). [Retrieved on 27 August 2021]. Retrieved from <URL: https://www.dbpia.co.kr/pdf/pdfView.do?nodeId=NODE00843814&mark=0&useDate=&bookmarkCnt=3&ipRange=N&accessgl=Y&language=ko_KR>.<br>    See pages 206-212. | 1-14 |
| A | JP 2012-223315 A (FUJIFILM CORP.) 15 November 2012 (2012-11-15)<br>    See paragraphs [0055]-[0074]; and figures 10-12. | 1-14 |
| A | JP 2020-077388 A (KOYU, Kai) 21 May 2020 (2020-05-21)<br>    See paragraphs [0022]-[0053]; and figures 6A-10. | 1-14 |

☑ Further documents are listed in the continuation of Box C.     ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
| --- | --- |
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **13 October 2021** | **13 October 2021** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

## INTERNATIONAL SEARCH REPORT

| International application No. |
|---|
| **PCT/KR2021/007532** |

### C. DOCUMENTS CONSIDERED TO BE RELEVANT

| Category* | Citation of document, with indication, where appropriate, of the relevant passages | Relevant to claim No. |
|---|---|---|
| PX | KR 10-2177951 B1 (DEEP BIO) 12 November 2020 (2020-11-12)<br>See paragraphs [0051]-[0070]; claims 1-14; and figures 1-4.<br>※ This document is a published earlier application that serves as a basis for claiming priority of the present international application. | 1-14 |

Form PCT/ISA/210 (second sheet) (July 2019)

**INTERNATIONAL SEARCH REPORT**
Information on patent family members

International application No.

**PCT/KR2021/007532**

| Patent document cited in search report | | | Publication date (day/month/year) | Patent family member(s) | | | Publication date (day/month/year) |
|---|---|---|---|---|---|---|---|
| KR | 10-2015-0020830 | A | 27 February 2015 | KR | 10-1442728 | B1 | 22 September 2014 |
| | | | | KR | 10-1514795 | B1 | 23 April 2015 |
| | | | | US | 2016-0117814 | A1 | 28 April 2016 |
| | | | | US | 9996918 | B2 | 12 June 2018 |
| | | | | WO | 2014-168350 | A1 | 16 October 2014 |
| JP | 2012-223315 | A | 15 November 2012 | EP | 2589340 | A1 | 08 May 2013 |
| | | | | EP | 2589340 | B1 | 18 June 2014 |
| | | | | JP | 5701138 | B2 | 15 April 2015 |
| | | | | US | 2013-0108133 | A1 | 02 May 2013 |
| | | | | US | 8634628 | B2 | 21 January 2014 |
| | | | | WO | 2012-144167 | A1 | 26 October 2012 |
| JP | 2020-077388 | A | 21 May 2020 | None | | | |
| KR | 10-2177951 | B1 | 12 November 2020 | None | | | |

Form PCT/ISA/210 (patent family annex) (July 2019)